# EUROPÄISCHE PATENTSCHRIFT

(11) **EP 0 929 659 B1**
(45) Veröffentlichungstag und Bekanntmachung des Hinweises auf die Patenterteilung: **19.05.2004**
(21) Anmeldenummer: 97938783.4
(22) Anmeldetag: 19.08.1997
(51) Int. Cl.: C12N 1/04, B65D 65/46, A01K 1/015, B01D 15/08

(54) **VERFAHREN ZUR HERSTELLUNG FESTER KÖRPER UND VERWENDUNGEN DERARTIGER KÖRPER**
PROCESS FOR PRODUCING SOLID BODIES AND USES OF SAID BODIES
PROCEDE DE PRODUCTION DE CORPS SOLIDES ET UTILISATION CORRESPONDANTE

(30) Priorität: 12.09.1996 DE 19637112
(43) Veröffentlichungstag der Anmeldung: 21.07.1999
(73) Patentinhaber: Kleespies, Matthias, 52353 Düren-Echtz (DE)
(72) Erfinder: Kleespies, Matthias, 52353 Düren-Echtz (DE)
(74) Vertreter: Castell, Klaus, Dr.
(86) Internationale Anmeldenummer: PCT/DE1997/001788
(87) Internationale Veröffentlichungsnummer: WO 1998/011201

(56) Entgegenhaltungen:
- FR-A- 1 330 904
- US-A- 4 229 440
- DATABASE WPI Section Ch, Week 8901 Derwent Publications Ltd., London, GB; Class D13, AN 89-003075 XP002052814 & JP 63 279 797 A (SUMITOMO HEAVY IND LTD) , 16.November 1988
- DATABASE WPI Section Ch, Week 9607 Derwent Publications Ltd., London, GB; Class D13, AN 96-067480 XP002052815 & KR 9 403 981 B (KIM N) , 10.Mai 1994

## Beschreibung

Die Erfindung betrifft ein Verfahren zur Herstellung fester Körper und Verwendungen derartiger fester Körper. Unter Körper werden sowohl verschieden große Partikel als auch beliebig geformte Körper verstanden.

Zur Herstellung fester Körper sind verschiedenartigste Verfahren je nach Ausgangsmaterial und beabsichtigter Verwendung der hergestellten Körper bekannt.

Der Erfindung liegt die Aufgabe zugrunde, ein weiteres Verfahren zur Herstellung fester Körper vorzustellen, das relativ anspruchslos in der Auswahl und Gewinnung der Ausgangsmaterialien ist und zu vielseitig einsetzbaren Produkten führt.

Nach dem erfindungsgemäßen Verfahren werden feste Körper hergestellt, indem Wasserkefirkulturen Nährstoffe zugegeben werden und die entstehende Masse getrocknet wird.

Wasserkefikulturen werden auch als japanische Meereskristalle bezeichnet und sind weißlich durchscheinende, knorpelige Klümpchen von unregelmäßiger Form und Größe. Die Klümpchen haben im Durchschnitt etwa einen Durchmesser von einem Zentimeter und unter dem Mikroskop haben sie ein kristallklares Aussehen.

Wasserkefir ist vom sog. Milchkefir zu unterscheiden. Während Milchkefir in Milch oder Molke wächst, gedeiht Wasserkefir in normalem Leitungswasser, dem Zucker und Nährstoffe zugegeben sind. Nur der Wasserkefir bildet die bekannten knorpeligen Klümpchen, die in der Nährstofflüssigkeit absinken. Dieser Wasserkefir ist aus der Herstellung unterschiedlicher Getränke bekannt. Der Wasserkefir ist eine Lebensgemeinschaft von Microorganismen, zu der u.a. verschiedene Milchsäurebakterien zählen.

Wasserkefirkulturen, die als sogenannte Kefirkörner erhältlich sind, stellen ein Gemisch aus homo- und heterofermentatativen Lactobacillusarten und mesophilen Milchsäure - Streptokokken und aus kohlendioxid- und alkoholproduzierenden Hefen sowie aus Essigsäurebakterien dar. Häufig wird jedoch nur ein Gemisch aus Milchsäurestreptokokken und Lactobacillusarten als Kefirkultur verwendet. Bei Wasserkefir verdoppeln sich die Microorganismen in etwa zwei Tagen.

Erfindungsgemäß werden diese Wasserkefirkulturen vermehrt und die dabei entstehende Masse, die ein Dextran darstellt, anschließend getrocknet, um feste Körper zu erhalten.

Wasserkefir hat den großen Vorteil, daß sich wasserunlösliche Dextrane bilden. Die zur Gewinnung des Dextrans notwendige nachgeschaltete Verfahrenstechnik ("Down Stream Processing") wird daher sehr stark vereinfacht, weil das Dextran in Form der gebildeten unlöslichen Partikel einfach mechanisch aus der Lösung entfernt werden kann und nicht aufwendig extrahiert werden muß.

Je nach verwendeter Nährstoffzusammensetzung sind den dextranbildenden Mikroorganismen Zusatzstoffe wie Stickstoff, Phospat, Vitamine oder Säurungsmittel beizugeben, um für ein optimales Milieu und eine optimale Versorgung der Mikroorganismen zu sorgen. Eine Steigerung der Dextranbildungsrate ist außerdem durch die Einstellung des optimalen pH-Werts und erhöhte Temperaturen im Bereich von 25°C bis 37°C zu erzielen.

Als Trockenverfahren eignet sich vor allem die Lufttrocknung, die Sprühtrocknung und die Vakuumtrocknung, wobei das Trocknungsverfahren auf die gewünschte Verwendung des Produkts abzustimmen ist. Je nach verwendeten Trocknungsverfahren können Partikel, Platten oder in Formen getrocknete Körper erzeugt werden. Außerdem sind die Partikelgrößen der Körper durch weitere Behandlungen, wie zum Beispiel das Vermahlen, einstellbar.

Ein besonderer Vorteil der erzeugten Körper liegt in deren biologischer Abbaubarkeit, die für viele Anwendungen besonders günstig ist. Durch Konservierungsstoffe kann die biologische Abbaubarkeit aber auch behindert oder auch nahezu vollständig vermieden werden.

Eine vorteilhaft Ausgestaltung des Verfahrens sieht vor, daß als Nährstoffe Abfallstoffe, insbesondere in der Tierhaltung nicht verfütterbare Abfallstoffe, verwendet werden. Als Abfallstoffe eignen sich vor allem Reststoffe aus der Zuckerindustrie und Zucker verarbeitenden Industrie und Stärkeherstellung oder -verarbeitung, die einen hohen Anteil an Zucker oder Stärke aufweisen. Auch Abfallstoffe aus der Papierindustrie sind als Nährstoffe geeignet, sofern die jeweiligen Enzyme oder weitere Mikroorganismen beigegeben werden, die einen Abbau der Zellulose oder der Stärke zu Mono- oder Disacchariden gewährleisten.

Die Form der entstehenden Körper ist dadurch variierbar, daß die Masse vor dem Trocknen in Formen gefüllt wird. Daneben ist auch eine Extrusion der vorgetrockneten Masse in Extrudern möglich. Dies erlaubt eine vorbestimmte Partikelgröße, die für viele Anwendungszwecke notwendig ist.

Eine besonders vorteilhafte Art des Trocknungsprozesses bietet die Gefriertrocknung. Wenn die Masse gefriergetrocknet wird, entstehen im wesentlichen gleichgroße Partikel, die eine poröse innere Struktur aufweisen. Diese Vergrößerung des Volumens bei vorgegebener Masse, erschließt den Körpern Anwendungsgebiete, in denen die Porösität der Körper von erhöhtem Nutzen ist.

Auch durch Zugabe von Treibmitteln zur Masse kann die Porösität der hergestellten Körper erhöht werden. Unter Treibmittel werden Stoffe verstanden, die unter der Einwirkung von Wärme oder Chemikalien Treibgase entwickeln. Hierbei sind vor allem Blähmittel zu erwähnen, die beispielsweise in der Kunststoff- und Baustoffindustrie zur Herstellung von Schaumstoffen verwendet werden, und Trieb- oder Teiglockerungsmittel, die in der Backwarenindustrie zur Lockerung von Teigen eingesetzt werden.

Die hergestellte Masse kann auch chemisch modifiziert werden. Dies erlaubt es sowohl aus der flüssigen bis knorpeligen Masse als auch der getrockneten Masse, z.B. eine verspinnbare Masse aus Polymeren herzustellen, ionische, dissoziierbare Gruppen anzuhängen, Moleküle zu vernetzen oder zu spalten. Die flüssige oder feste ggf. chemisch modifizierte Masse kann auch in Haarpflegemitteln oder als Haarpflegemittel verwendet werden.

Die beschriebenen Körper können für verschiedene Einsatzbereiche verwendet werden. Besonders wichtig ist der Einsatz der Körper als Packoder Packhilfsmittel. In Form von Chips, Hohlkugeln, oder Halbkugeln oder auch als an die Ware angepaßte Form können sie bisher verwendete Pack- oder Packhilfsmittel aus Kunststoffen ersetzen. Dies führt zu dem Vorteil, daß die erfindungsgemäßen Körper zusammen mit den übrigen Packstoffen kompostierbar oder zumindest biologisch abbaubar sind. Die Weiterverwendung der Pack- oder Packhilfsmittel wird daher durch den Einsatz der erfindungsgemäßen Körper stark vereinfacht.

Ein weiterer wichtiger Verwendungsbereich der erfindungsgemäßen Körper liegt in der Verwendung als Bodenverbesserung. Hierbei dienen die erfindungsgemäßen Körper als Partikel der Lockerung des Bodens und sie erhöhen die Fähigkeit des Bodens, Wasser zu binden und zu speichern.

Je nach verwendeten Ausgangsmaterialien sind die erfindungsgemäßen Körper auch als Lebens- oder Futtermittel einsetzbar. Hierbei ist vor allem auch die Beimischung derartiger Körper zu herkömmlichen Futtermitteln wichtig. Sowohl die Konsistenz, als auch die Zusammensetzung der Körper prädestiniert sie für den Einsatz als Kraftfutter für die Rinder- und Schweinehaltung.

Die Körper sind auch für einen Einsatz als Trocknungsmittel oder Absorbens hervorragend geeignet, da sie aufgrund ihrer Zusammensetzung und physikalischen Struktur besonders hygroskopisch sind. Insbesondere ein feines Vermahlen der Körper führt zu einem Superabsorbens, das bei gleichzeitiger biologischer Abbaubarkeit hervorragend für die Herstellung von Windeln geeignet ist.

Ein weiterer Anwendungsbereich der Körper liegt in der Bauindustrie, wo sie als Dämmaterial in Partikelform oder als Platten und Formkörper einsetzbar sind.

Darüberhinaus sind die Körper in Partikelform auch als Unterlage zur Tierhaltung, insbesondere als Katzenstreu, geeignet.

Die Verwendung der festen Körper als feste Elektrolyte oder als Elektrolyte in wässriger Lösung erschließt eine Vielzahl weiterer Anwendungen z.B. für Sensoren, Batterien, Brennstoffzellen und elektronische Schaltkreise.

Letztlich sind die erzeugten Körper auch als stationäre Phase bei der Chromatographie, insbesondere bei der HPLC einsetzbar.

Ein Ausführungsbeispiel der Erfindung wird im folgenden beschrieben.

Flüssige, zuckerhaltige Reststoffe aus der Zuckerindustrie werden in größeren Becken mit Wasserkefir versetzt. Der pH-Wert wird auf ca. pH 5 eingestellt und außerdem werden der Lösung Stickstoff, Phosphat und Vitamine zugeführt, um eine optimale Versorgung der Mikroorganismen zu gewährleisten. Durch eine Verdopplung der Mikroorganismen in etwa zwei Tagen entstehen schleimartige Klumpen, die sich am Beckenboden anreichern und dann gefriergetrocknet werden. Bei der Gefriergetrocknung entstehen poröse Körner mit einem Durchmesser von etwa 4-5 mm, die anschließend z.B. als Mittel zur Bodenverbesserung, als Lebens- oder Futtermittel oder auch als Trocknungsmittel einsetzbar sind. Vor allem die größeren Partikel eignen sich auch als Pack- oder Packhilfsmittel zur Verpackung empfindlicher Güter.

## Patentansprüche

1. Verfahren zur Herstellung fester Körper, **dadurch gekennzeichnet, daß** Wasserkefirkulturen Nährstoffe zugegeben werden und die entstehende Masse getrocknet wird.

2. Verfahren nach Anspruch 1, **dadurch gekennzeichnet, daß** als Nährstoffe Abfallstoffe, insbesondere in der Tierhaltung nicht verfütterbare Abfallstoffe, verwendet werden.

3. Verfahren nach einem der vorhergehenden Ansprüche, **dadurch gekennzeichnet, daß** die Masse vor dem Trocknen in Formen gefüllt wird.

4. Verfahren nach einem der vorhergehenden Ansprüche, **dadurch gekennzeichnet, daß** die Masse gefriergetrocknet wird.

5. Verfahren nach einem der vorhergehenden Ansprüche, **dadurch gekennzeichnet, daß** der Masse ein Treibmittel beigegeben wird.

6. Verfahren nach einem der vorhergehenden Ansprüche, **dadurch gekennzeichnet, daß** die Masse chemisch modifiziert wird.

7. Verwendung der nach einem der Ansprüche 1 bis 6 hergestellten festen Körper als Pack- oder Packhilfsmittel.

8. Verwendung der nach einem der Ansprüche 1 bis 6 hergestellten festen Körper als Dämmaterial.

9. Verwendung der nach einem der Ansprüche 1 bis 6 hergestellten festen Körper als Unterlage zur Tierhaltung, insbesondere als Katzenstreu.

10. Verwendung der nach einem der Ansprüche 1 bis 6 hergestellten festen Körper als Elektrolyt, insbesondere Polyelektrolyt.

11. Verwendung der nach einem der Ansprüche 1 bis 6 hergestellten festen Körper als stationäre Phase bei der Chromatographie.

## Claims

1. A method for manufacturing solid bodies, **characterized in that** nutrients are added to water kefir cultures and that the resulting mass is dried.

2. The method according to claim 1, **characterized in that** waste substances, more specifically waste substances that cannot be used as animal feed, are used as nutrients.

3. The method according to one of the afore-mentioned claims, **characterized in that** the mass is poured into moulds before drying.

4. The method according to one of the afore-mentioned claims, **characterized in that** the mass is freeze dried.

5. The method according to one of the afore-mentioned claims, **characterized in that a** leavening agent is added to the mass.

6. The method according to one of the afore-mentioned claims, **characterized in that** the mass is modified chemically.

7. Use of the solid bodies manufactured according to one of the claims 1 to 6 as a packaging material or as an auxiliary packaging material.

8. Use of the solid bodies manufactured according to one of the claims 1 to 6 as an insulating material.

9. Use of the solid bodies manufactured according to one of the claims 1 to 6 as a bedding for animals, more specifically as cat litter.

10. Use of the solid bodies manufactured according to one of the claims 1 to 6 as an electrolyte, more specifically as a polyelectrolyte.

11. Use of the solid bodies manufactured according to one of the claims 1 to 6 as the stationary phase in chromatography.

## Revendications

1. Procédé de fabrication de corps solides, **caractérisé en ce que** des substances nutritives sont ajoutées à des cultures de kéfir d'eau et que la masse obtenue est séchée.

2. Procédé selon la revendication 1, **caractérisé en ce que** des déchets, notamment des déchets qui ne peuvent servir d'aliments pour animaux dans l'élevage, sont utilisés en tant que substances nutritives.

3. Procédé selon l'une quelconque des revendications précédentes, **caractérisé en ce que** la masse est versée dans des moules avant le séchage.

4. Procédé selon l'une quelconque des revendications précédentes, **caractérisé en ce que** la masse est lyophilisée.

5. Procédé selon l'une quelconque des revendications précédentes, **caractérisé en ce qu'** une levure est ajoutée à la masse.

6. Procédé selon l'une quelconque des revendications précédentes, **caractérisé en ce que** la masse est modifiée chimiquement.

7. Utilisation des corps solides fabriqués selon l'une quelconque des revendications 1 à 6 en tant que matériel ou accessoires pour emballage.

8. Utilisation des corps solides fabriqués selon l'une quelconque des revendications 1 à 6 en tant que matériel isolant.

9. Utilisation des corps solides fabriqués selon l'une quelconque des revendications 1 à 6 en tant que litière dans l'élevage, notamment en tant que litière pour chats.

10. Utilisation des corps solides fabriqués selon l'une quelconque des revendications 1 à 6 en tant qu'électrolyte, notamment en tant que polyélectrolyte.

11. Utilisation des corps solides fabriqués selon l'une quelconque des revendications 1 à 6 en tant que phase fixe en chromatographie.
